# EUROPÄISCHE PATENTSCHRIFT

(11) **EP 0 888 328 B1**
(45) Veröffentlichungstag und Bekanntmachung des Hinweises auf die Patenterteilung: **25.07.2001**
(21) Anmeldenummer: 97908197.3
(22) Anmeldetag: 07.03.1997
(51) Int. Cl.: C07D 317/22, C09K 19/58

(54) **CHIRALE VERBINDUNGEN UND IHRE ANWENDUNG ALS DOTIERSTOFFE IN FLÜSSIGKRISTALLEN**
CHIRAL COMPOUNDS AND THEIR USE AS DOPING AGENTS IN LIQUID CRYSTALS
COMPOSES CHIRAUX ET LEUR UTILISATION EN TANT QU'AGENTS DOPANTS DANS DES CRISTAUX LIQUIDES

(30) Priorität: 21.03.1996 DE 19611101
(43) Veröffentlichungstag der Anmeldung: 07.01.1999
(73) Patentinhaber: BASF AKTIENGESELLSCHAFT, 67056 Ludwigshafen (DE)
(72) Erfinder: MEYER, Frank, D-68165 Mannheim (DE); SIEMENSMEYER, Karl, D-67227 Frankenthal (DE); KUBALL, Hans-Georg, D-67705 Stelzenberg (DE); WEISS, Bernhard, D-67663 Kaiserslautern (DE); SEEBACH, Dieter, CH-8044 Zürich (CH)
(86) Internationale Anmeldenummer: EP9701163
(87) Internationale Veröffentlichungsnummer: WO9734886

(56) Entgegenhaltungen:
- EP-A- 0 233 602
- EP-A- 0 387 196
- DE-A- 4 342 280
- US-A- 5 498 367
- HELVETICA CHIMICA ACTA, Bd. 79, Nr. 9, 18.September 1996, BASEL CH, Seiten 1710-1740, XP002032705 DIETER SEEBACH ET AL.: "Polymer- and Dendrimer-Bound Ti-TADDOLates in Catalytic (and Stoichiometric) Enantioselective Reactions: Are Pentacoordinate Cationic Ti Complexes the Catalytically Active Species?"
- JOURNAL OF THE AMERICAN CHEMICAL SOCIETY, Bd. 114, Nr. 7, 25.März 1992, DC US, Seiten 2321-2336, XP002032706 ANDREAS HAFNER ET AL. : "Enantioselective Allyltitanation of Aldehydes with Cyclo- pentadienyldialkoxyallytitanium Complexes"

## Beschreibung

Gegenstand der vorliegenden Erfindung sind chirale Verbindungen der allgemeinen Formel I 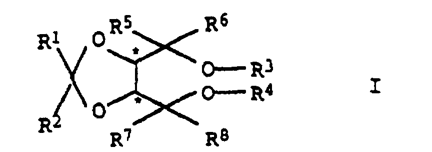 wobei die Variablen die folgende Bedeutung haben:
- R¹,R²: Wasserstoff oder aromatische, aliphatische oder araliphatische Reste mit 1 bis 40 C-Atomen,
- R³,R⁴: aromatische, aliphatische oder araliphatische Reste mit 1 bis 40 C-Atomen,
- R⁵,R⁶,R⁷,R⁸: aromatische oder aliphatische Reste mit 1 bis 20 C-Atomen,
mit der Maßgabe, daß mindestens einer der Reste R¹ bis R⁸ eine polymerisierbare Gruppe enthält.

Weiterhin betrifft die Erfindung die Verwendung von chiralen Verbindungen der allgemeinen Formel Ia 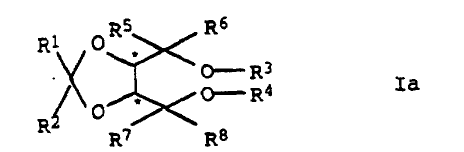 wobei die Variablen die folgende Bedeutung haben:
- R¹,R²,R^{3,}R⁴: Wasserstoff oder aromatische, aliphatische oder araliphatische Reste mit 1 bis 40 C-Atomen,
- R⁵,R⁶,R⁷,R⁸: aromatische oder aliphatische Reste mit 1 bis 20 C-Atomen,
außerdem flüssigkristalline Zusammensetzungen, welche die chiralen Verbindungen enthalten, die Verwendung dieser flüssigkristallinen Zusammensetzungen zur Beschichtung von Substraten und zur Herstellung von Interferenzpigmenten sowie die Verwendung der Interferenzpigmente in Druckfarben und Lacken.

Chirale Verbindungen der Formel Ia, in denen R³ und R⁴ Wasserstoff und R⁵ und R⁶ Arylreste bedeuten sind unter der Bezeichnung TADDOLe (α, α, α', α'-Tetraaryl-1,3-dioxolan-4,5-dimethanol) seit langem bekannt und werden z.B. als Katalysatoren für enantioseleketive Synthesen verwendet. (vgl. Dahinden et al. in "Encyclopedia of Reagents for Organic Synthesis", Paquette, L.A., Ed.; John Wiley & Sons, Chichester, 1995).

Flüssigkristalline Phasen, welche chirale Verbindungen enthalten, weisen oft bemerkenswerte Eigenschaften auf. So können sich z.B. durch die Dotierung einer nematischen flüssigkristallinen Phase mit einer chiralen Verbindung cholesterische Phasen bilden, in welcher die Moleküle der flüssigkristallinen Verbindung helixartig angeordnet sind. Solche cholesterischen Phasen zeigen oft farbige Interferenzeffekte durch Selektivreflexion von Licht bestimmter Wellenlänge an der helixartigen Flüssigkristallstruktur. Diese farbigen cholesterischen Phasen lassen sich z.B. durch Abkühlung unter die Glastemperatur oder Einbau in polymere Netzwerke fixieren, wodurch eine Verwendung in farbigen Beschichtungen oder als Interferenzpigmente ermöglicht wird.

Als chirale Dotierstoffe für flüssigkristalline Phasen sind zahlreiche Verbindungen bekannt (z.B. aus DE-A 4342280).

Die US-A-5,498,367 beschreibt chirale Dotierstoffe für Flüssigkristalle, die sich von 1,3-Dioxolan-4,5-dicarbonsäuren ableiten. Geeignete Dotierstoffe sollten eine hohe Verdrillungsfähigkeit besitzen, so daß geringe Mengen des Dotierstoffs zur Induktion der helikalen Struktur ausreichen. Außerdem sollten die chiralen Dotierstoffe eine gute Kompatibilität zu den flüssigkristallinen Verbindungen zeigen, so daß eine effektive Wechselwirkung zwischen diesen Komponenten ermöglicht wird. Hinsichtlich dieser Eigenschaften lassen viele der bekannten chiralen Dotierstoffe noch zu wünschen übrig. Außerdem eignen sich viele chirale Verbindungen nur begrenzt für polymere cholesterische Flüssigkristallsystemen, da sie nicht kovalent in polymere Netzwerke eingebaut werden können.

Aufgabe der vorliegenden Erfindung war es daher, chirale Verbindungen zur Verfügung zu stellen, die sich auf Grund ihre Kompatibilität mit flüssigkristallinen Verbindungen und ihre Verdrillungsfähigkeit gut als Dotierstoffe für Flüssigkristalle eignen.

Demgemäß wurden die eingangs erwähnten chiralen Verbindungen der Formeln I und Ia gefunden.

In den allgemeinen Formeln I und Ia sind die Reste R⁵, R⁶, R⁷ und R⁸ aromatische oder aliphatische Reste mit 1 bis 20 C-Atomen. Vorzugsweise sind diese Reste sterisch anspruchsvolle Reste, da ihr Raumbedarf besonderen Einfluß auf die Verdrillungsfähigkeit der Verbindungen hat. So ist vorzugsweise mindestens einer der Reste in den Restepaaren R⁵, R⁶ bzw. R⁷, R⁸ ein verzweigter oder zyklischer aliphatischer Rest mit 6-20 C-Atomen. Der räumliche Anspruch der Substituenten ist dabei wichtiger als die chemische Zusammensetzung. So kommen z.B. neben reinen Kohlenwasserstoffresten auch durch Sauerstoff, Schwefel, Imino oder C₁-C₄-Alkyliminogruppen unterbrochene Alkyl- oder Cycloalkylgruppen in Betracht. Unter den Kohlenwasserstoffresten sind beispielsweise zu nennen: Methyl, Ethyl, Isopropyl, n-Propyl, n-Butyl, Isobutyl, sec.-Butyl, tert.-Butyl, n-Pentyl, Isopentyl, Neopentyl, tert.-Pentyl, sowie vorzugsweise verzweigtes Hexyl, Heptyl, Octyl, Nonyl, Decyl, Undecyl, Dodecyl, Tridecyl, Tetradecyl, Hexadecyl, Octadecyl und Eicosyl. Weiterhin kommen cycloaliphatische Reste wie Cyclopentyl, Cyclohexyl, Cycloheptyl oder Decahydronaphthyl in Betracht.

Besonders bevorzugt sind als R⁵, R⁶, R⁷ und R⁸ aromatische Reste zu nennen, insbesondere Phenyl- und Naphtylreste. Die Phenyl- und Naphtylreste sind aufgrund der einfacheren Zugänglichkeit vorzugsweise unsubstituiert, können jedoch auch z.B. durch C₁-C₄-Alkylreste aus der oben genannten Gruppe substituiert sein. Besonders bevorzugte Substituenten für die Phenyl- oder Naphtylreste sind Vinyl- und Vinyloxyreste, da über diese polymerisierbaren Gruppen eine Fixierung der chiralen Verbindungen in polymeren Flüssigkristallen möglich ist. Weiter kommen als Substituenten für die Phenyl- oder Napthylreste Hydroxygruppen in Betracht, welche beispielsweise mit Acrylsäure oder Methacrylsäure zu den entsprechen Estern umgesetzt werden können und so die Einführung einer polymerisierbaren Gruppe nach der Synthese einer Verbindung I bzw. Ia ermöglichen.

Die Reste R¹, R², R³ und R⁴ haben weniger Einfluß auf die Verdrillungsfähigkeit als die Reste R⁵, R⁶, R⁷ und R⁸, sondern sie beeinflussen vor allem die Kompatibilität mit den flüssigkristallinen Wirtssystemen. Aus diesem Grund sind ihre chemischen Eigenschaften wichtiger als ihre räumliche Ausdehnung. Vorteilhaft ist oft eine Ähnlichkeit zumindestens einiger Reste R¹ bis R⁴ mit den chemischen Strukturen der flüssigkristallinen Verbindungen. Dadurch werden eine gute Löslichkeit in der Flüssigkristallphase und intensive Wechselwirkungen erreicht, was wiederum zu effektiver Verdrillung der Flüssigkristallphase führt, Wegen der Vielzahl möglicher flüssigkristalliner Wirtssysteme kommt daher auch eine Vielzahl verschiedener Strukturen für die Reste R¹ bis R⁴ in Betracht.

Als Reste R¹ und R² kommen neben Wasserstoff sehr unterschiedliche aromatische, aliphatische oder araliphatische Reste in Betracht. Je nachdem, ob das Dioxolanringsystem, an dessen 2-Position die Reste R¹ und R² stehen, durch Acetalisierung oder Ketalisierung gebildet wird, können beide Reste organische Reste sein oder es kann einer der Reste Wasserstoff bedeuten, wobei diese letztere Möglichkeit bevorzugt ist. Als aliphatische Reste R¹ und/oder R² kommen beispielsweise die C₁-C₂₀-Alkylreste in Betracht, wie sie für R⁵ bis R⁸ genannt wurden. In diesen Alkylresten können nicht benachbarte CH₂-Gruppen durch Sauerstoff, Schwefel oder C₁-C₄-Alkylimino ersetzt sein. Besonders zu nennen von diesen einfachen aliphatischen Resten ist der Methylrest.

Weiterhin kommen als Reste R¹ und/oder R² aromatische Reste wie Phenyl oder substituierte Phenylreste in Betracht. Die Substituenten der Phenylreste können z.B. die oben genannten gegebenenfalls durch Sauerstoff, Schwefel oder C₁-C₄-Alkylimino unterbrochenen C₁-C₂₀-Alkylreste, entsprechende C₁-C₂₀-Alkoxyreste oder Halogen, wie Chlor oder Brom, oder der Cyanorest sein. Bevorzugte Substituenten sind auch hier polymerisierbare Reste, wie Vinyl, Vinyloxy, Acryloxy oder Methacryloxy.

Bevorzugte Reste R¹ und R² sind solche der allgemeinen Formel

-(A¹-Y²)ₘ-(T-Y³)ₘ-(A²-Y⁴)ₒ-Z

in der die Variablen die folgende Bedeutung haben:
- Y²,Y³,Y⁴: eine kovalente Einfachbindung, Sauerstoff, Schwefel, -CO-O-, -O-CO-, -O-CO-O-, -CO-NR⁹- oder -NR⁹-CO-, wobei diese Brückenglieder gleich oder verschieden sein können,
- A¹,A²: gleiche oder verschiedene Alkylenreste mit 2 bis 12 C-Atomen, in denen nichtendständige nichtbenachbarte CH₂-Gruppen durch Sauerstoff ersetzt sein können,
- T: gleiche oder verschiedene aliphatische oder aromatische Ringe, welche bis zu drei Heteroatome drei enthalten können,
- R⁹: Wasserstoff oder C₁-C₄-Alkyl,
- m,o: 0 oder 1,
- n: 0, 1, 2, 3 oder 4 und
- Z: Wasserstoff oder ein polymerisierbarer Rest.

Bevorzugte Brückenglieder Y², Y³ und Y⁴ sind neben der direkten Bindung -CO-O-, -O-CO- und -O-CO-O-.

Als Spacer A¹ und A² kommen beispielsweise die Gruppen
- (CH₂)ᵣ- oder - (CH₂CH₂O)ₛ-CH₂CH₂-
in Betracht, wobei r 1 bis 12 und s 1 bis 3 bedeuten.

Die Reste T können beispielsweise durch Fluor, Chlor, Brom, Cyan, Hydroxy oder Nitro substituierte Ringsysteme sein. Bevorzugte Reste T sind:

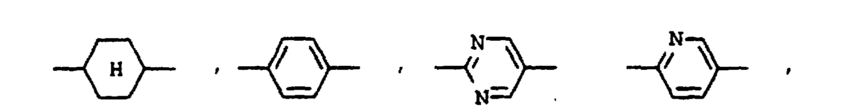

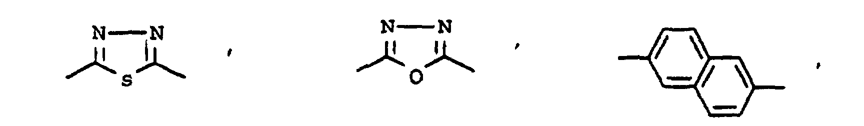

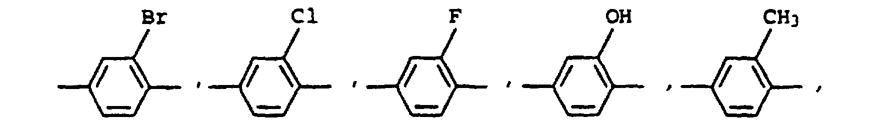

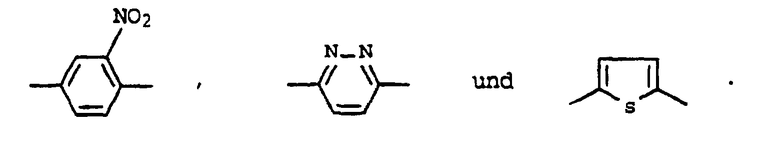

Besonders bevorzugt sind die folgenden Molekülteile (T-Y³)ₙ:

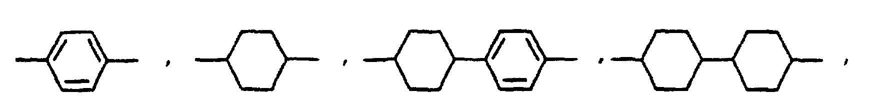

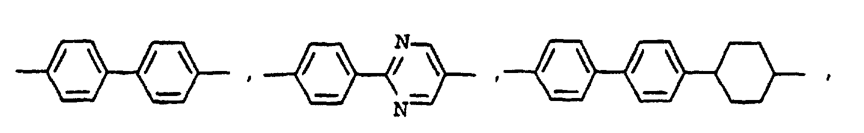

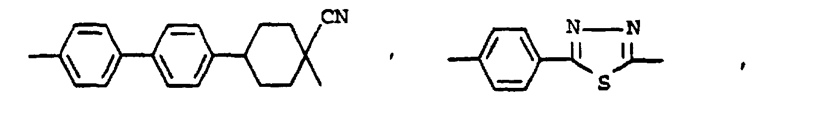

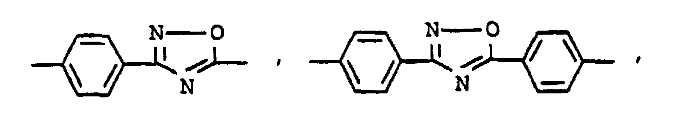

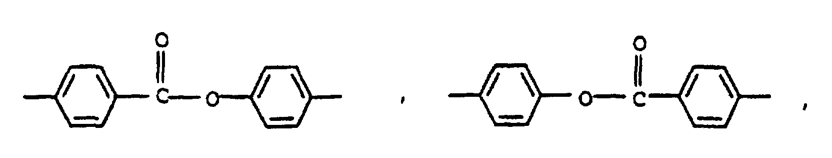

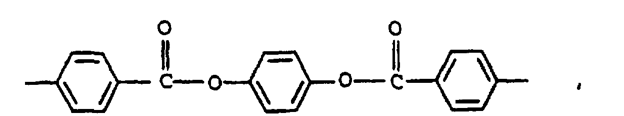

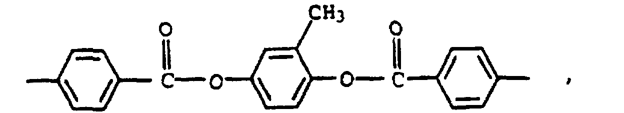

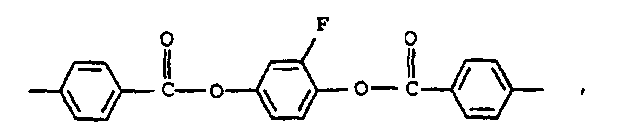

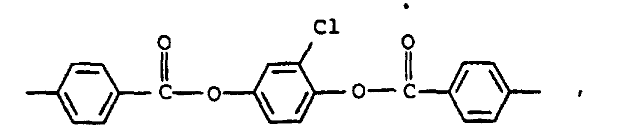

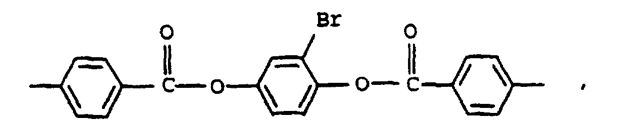

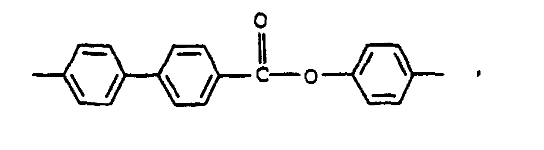

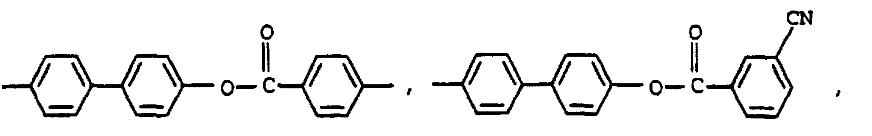

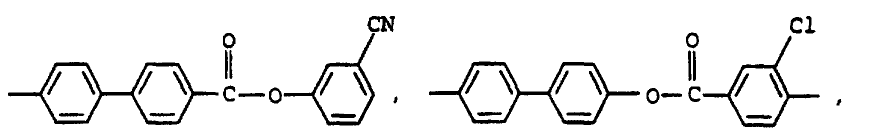

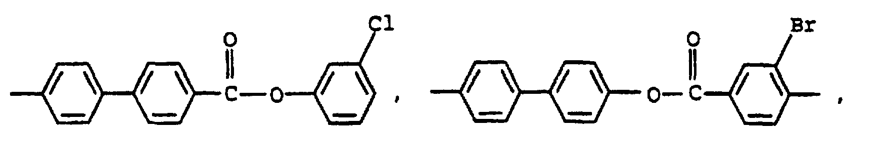

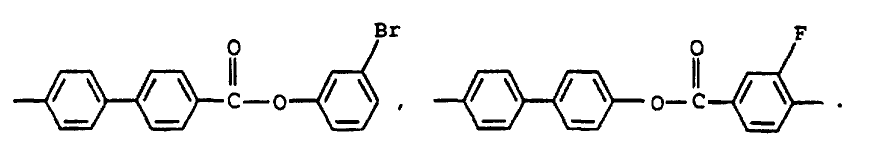

Als R⁹ kommen neben Wasserstoff Methyl, Ethyl, Propyl und Butyl in Betracht.
m und o sind bevorzugt 1, n ist vorzugsweise 2 oder 3

Als Reste Z kommen neben Wasserstoff auch polymerisierbare Reste in Betracht. Bevorzugt polymerisierbare Reste sind beispielsweise H₂C=CH-, HC≡C-, 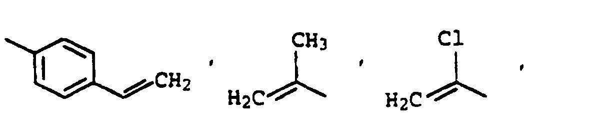 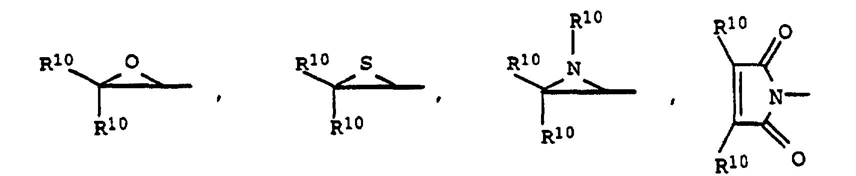 ― N = C = O , ― N = C = S, ― O ― C ≡ N,
― COOH, ― OH oder ― NH₂, wobei die Reste R¹⁰ gleich oder verschieden sein können und Wasserstoff oder C₁-C₄-Alkyl wie Methyl, Ethyl, Propyl, Isopropyl, n-Butyl, iso-Butyl, sec.-Butyl oder tert.-Butyl bedeuten.

Dabei ist zu beachten, daß bei den über Heteroatome gebundenen polymerisierbaren Resten die Bindung an ein Kohlenstoffatom erfolgen muß, d.h. Y⁴ muß in diesen Fallen eine direkte Bindung sein.

Bevorzugte Gruppierungen Y⁴-Z sind Acryloxy, Methacryloxy, Vinyl, Vinyloxy, Styryl und Styryloxy.

Die Reste R³ und R⁴ sind, insbesondere, wenn R⁵ bis R⁸ sehr sperrige Reste bedeuten, bevorzugt Wasserstoff. Um die Kompatibilität mit flüasigkristallinen Verbindungen zu erhöhen, können die Reste R³ und/oder R⁴ aber auch Reste mit den für R¹ und R² genannten Strukturmerkmalen bedeuten. Dabei gelten im wesentlichen die gleichen Bevorzugungen, wie sie für R¹ und R² bereits genannt sind. Insbesondere sind als R³ und R⁴ Reste der allgemeinen Formel

-Y¹- (A¹-Y²)ₘ-(T-Y³)ₙ-(A²-Y⁴)ₒ-Z

geeignet, in der die Variablen die oben genannten Bedeutungen haben und Y¹ eine kovalente Einfachbindung, -CO- oder -CO-O- bedeutet.

Für diese Variablen gelten im wesentlichen die oben genannten Bevorzugungen.

Die Herstellung von TADDOLen ist an sich bekannt, z.B, von D. Seebach et al, J. Org. Chem. 60, 1788 (1995). Zur Herstellung der Verbindungen I und Ia können die gleichen Synthesewege wie für die bekannten TADDOLe gewählt werden. Im allgemeinen geht man von Weinsauremethylestern aus. Im ersten Schritt wird üblicherweise der 1,3-Dioxolanring durch Umsetzung mit dem gewünschten Aldehyd oder Keton der Formel R¹-CO-R² gebildet.

Die bevorzugten Molekülteile der Formel

- (A¹-Y²)ₘ- (T-Y³)ₙ-(A²-Y⁴)ₒ-Z

können in bekannter Weise durch übliche, dem Fachmann vertraute Kondensationsreaktionen der Elemente A¹/A² mit T-(Y³-T)ₙ₋₁ bzw. Z erhalten werden, wobei die übrigen Brückenglieder Y² und Y⁴ gebildet werden. Derartige Reaktionen sind beispielsweise in der älteren deutschen Patentanmeldung 19532408.0 beschrieben.

Die Substituenten R⁵, R⁶, R⁷ und R⁸ können dann durch Umsetzung mit Grignardverbindungen der Formel R⁵MgBr erhalten werden. Sollen R⁵ bis R⁸ unterschiedliche Reste sein, setzt man zunächst mit einer geringeren Menge (z.B. Molverhältnis 4:1) Grignardverbindungen um, reinigt das monosubstituierte Produkt auf und setzt anschließend sukzessive mit den anderen Grignardverbindungen um. Nach den Grignardreaktionen erhält man so die Verbindungen I oder Ia, in denen R³ und R⁴ Wasserstoff bedeuten.

Andere Reste R³ und/oder R⁴ erhält man beispielsweise durch bekannte Methoden der Ethersynthese, z.B. durch Umsetzung mit Verbindungen Halogen-R³ oder durch Umsetzungen mit Säurehalogeniden oder Halogenkohlensäureestern, je nachdem, ob in den bevorzugten Resten der Formel -Y¹-(A¹-Y²)ₘ-(T-Y³)ₙ-(A²-Y⁴)ₒ-Z die Variable Y¹ eine kovalente Einfachbindung, eine Carbonylgruppe oder eine Oxycarbonylgruppe bedeutet.

Die polymerisierbaren Gruppen der Verbindungen I oder Ia können entweder im Rahmen der oben angegebenen Verfahrensschritte durch Umsetzung mit Verbindungen, die bereits polymerisierbare Substituenten tragen, eingeführt werden, oder sie können nach diesen Syntheseschritten z.B. über Veresterungen freier Hydroxylgruppen in den entsprechenden Resten z.B. mit Acrylsäure oder Methacrylsäure eingeführt werden.

Erfindungsgemäße flüssigkristalline Zusammensetzungen enthalten eine oder mehrere der chiralen Verbindungen I oder Ia als Dotierstoffe, üblicherweise in Konzentrationen zwischen 0,1 und 10 Gew.-%, bezogen auf die Gesamtmenge des Flüssigkristalls. Die Konzentration wird dabei so gewählt, daß der gewünschte Interferenzfarbton entsteht. Höhere Konzentrationen verschieben den Farbton in den blauen Bereich, niedrigere in dem roten Bereich.

Besonders geeignete flüssigkristalline Zusammensetzungen sind solche, die als achirale flüssigkristalline Verbindungen polymerisierbare Verbindungen enthalten, da bei solchen Zusammensetzungen der cholesterisch flüssigkristalline Ordnungszustand durch Polymerisation fixiert werden kann. Geeignete polymerisierbare flüssigkristalline Verbindungen sind beispielsweise in WO 93/05436, EP-A 261 712 und in der älteren deutschen Patentanmeldung 19532408.0 beschrieben. Besonders stabile Systeme sind solche, in denen auch der chirale Dotierstoff I oder Ia mindestens eine polymerisierbare Gruppe enthält, da auf diese Weise ein Herausdiffundieren des Dotierstoffs aus der flüssigkristallin geordneten Phase verhindert wird.

Die genannten flüssigkristallinen Zusammensetzungen lassen sich vorteilhaft zur Beschichtung von Substraten verwenden. Geeignete Substrate sind beispielsweise Metalloberflächen, Kunststoffoberflächen, Glas- oder Keramikoberflächen oder Folien.

Weiterhin lassen sich die erfindungsgemäßen flüssigkristallinen Zusammensetzungen zur Herstellung von Interferenzpigmenten verwenden. Beispielsweise werden dazu die vorzugsweise polymerisierbaren Zusammensetzungen auf ein Substrat, vorzugsweise Glas oder eine glatte Folie, aufgetragen, gegebenenfalls durch Rakeln oder andere physikalische Einflüsse orientiert und dann polymerisiert. Der polymerisierte Flüssigkristallfilm wird dann vom Substrat abgelöst und auf Pigmentpartikelgröße zerkleinert. Weitere Einzelheiten zu geeigneten Pigmentherstellungsverfahren sind in den älteren deutschen Patentanmeldungen 19 602 795.0 und 19 602 848.5 beschrieben. Die dabei entstehenden überwiegend plättchenförmigen Pigmente können als Effektpigmente mit blickwinkelabhängigem Farbeindruck in Druckfarben und Lacken verwendet werden.

### Beispiele

### Beispiel 1

### Herstellung von (4 S-trans)-2,2-Dimethyl-α,α,α',α',-tetra(2-naphthyl)-α,α',-di-(Acryloyloxymethyl)-1,3-dioxolan

0,5 g (0,75 mmol) (4 S-trans)-2,2-Dimethyl-α,α,α',α',-tetra(2-naphthyl)-1,3-dioxolan-4,5-dimethanol wurden in 40 ml Toluol gelöst. Zu der Lösung wurden 0,15 g (1,5 mmol) Triethylamin und 0,34 g (3,75 mmol) Acrylsäurechlorid gegeben. Die Lösung wurde 14 Stunden bei 20°C gerührt. Anschließend wurde mit Wasser und dann mit verdünnter Salzsäure gewaschen, der entstandene Niederschlag wurde abgesaugt und die organische Lösung über Natriumsulfat getrocknet. Die Reinigung erfolgte säulenchromatographisch.

Ausbeute: 0,36 g
¹H-NMR (CDCl₃): 0,95(s); 1,1(s); 4,1 (m); 5,05 (d); 5,6 (d); 5.95 (m); 6,1 (dd); 7,3-8,1 (m).

Helical Twisting Power (HTP): 27,3 µm⁻¹

(Die HTP wurde nach der von H. Finkelmann und H. Stegemeyer in Ber. Bunsengesellschaft Phys. Chem. 78, 869(1974) angegebenen Methode bestimmt. Al nematische Wirtsphase diente die Flüssigkristallmischung ZLI 1840® der Fa. Merck, Darmstadt, Germany.)

### Beispiel 2

Herstellung von (4 S-trans)-2,2-Dimethyl-α,α,α',α',-tetra(2-naphthyl)-α,α',-di- (acryloyloxybutyloxycarbonyloxy)-1,3-dioxolan.

Analog zu Beispiel 1 wurden 0,5 g (0,75 mmol) (4 S-trans)-2,2-Dimethyl-α,α,α',α',-tetra(2-naphthyl)-1,3-dioxolan-4,5-dimethanol mit 0,77 g (3,75 mmol) 4-(Acryloyloxy)-butyloxychloroformiat umgesetzt und aufgearbeitet.

Ausbeute: 0,15 g
¹H-NMR (CDCl₃): 0,95 (s); 1,0 (s); 1,5 (bs); 3,87-3,90 (m); 4,05 (m); 5,05 (d); 5,6 (d); 5,95 (m); 6,1 (dd); 8,1-7,3 (m).

Helical Twisting Power (HTP): 37,5 µm⁻¹

## Patentansprüche

1. Chirale Verbindungen der allgemeinen Formel I 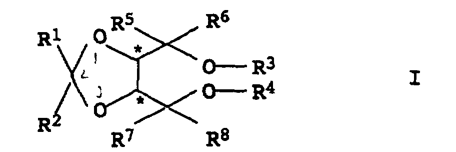 wobei die Variablen die folgende Bedeutung haben:
R¹,R² Wasserstoff oder aromatische, aliphatische oder araliphatische Reste mit 1 bis 40 C-Atomen,
R³,R⁴ aromatische, aliphatische oder araliphatische Reste mit 1 bis 40 C-Atomen,
R⁵,R⁶,R⁷,R⁸ aromatische oder aliphatische Reste mit 1 bis 20 C-Atomen,
mit der Maßgabe, daß mindestens einer der Reste R¹ bis R⁸ eine polymerisierbare Gruppe enthält.

2. Chirale Verbindungen I nach Anspruch 1, in denen R⁵, R⁶, R⁷ und R⁸ unsubstituierte oder mit Vinyl- oder Vinyloxygruppen substituierte Phenyl- oder Naphthylreste sind.

3. Chirale Verbindungen I nach Anspruch 1 oder 2, in denen R³ und R⁴ Reste der allgemeinen Formel
-Y¹-(A¹-Y²)ₘ-(T-Y³)ₙ-(A²-Y⁴)ₒ-Z
sind, in der die Variablen die folgende Bedeutung haben:
Y¹ eine kovalente Einfachbindung, -CO- oder -CO-O-,
Y²,Y³,Y⁴ eine kovalente Einfachbindung, Sauerstoff, Schwefel, -CO-O-, -O-CO-, -O-CO-O-, -CO-NR⁹- oder -NR⁹-CO-, wobei diese Brückenglieder gleich oder verschieden sein können,
A¹,A² gleiche oder verschiedene Alkylenreste mit 2 bis 12 C-Atomen, in denen nichtendständige nichtbenachbarte CH₂-Gruppen durch Sauerstoff ersetzt sein können,
T gleiche oder verschiedene aliphatische oder aromatische Ringe, welche bis zu drei Heteroatome enthalten können,
R⁹ Wasserstoff oder C₁-C₄-Alkyl,
m,o 0 oder 1,
n 0, 1, 2, 3 oder 4 und
Z Wasserstoff oder ein polymerisierbarer Rest.

4. Chirale Verbindungen I nach den Ansprüchen 1 bis 3, in denen R¹ und R² unabhängig voneinander Wasserstoff oder Reste der allgemeinen Formel
-(A¹-Y²)ₘ-(T-Y³)ₙ-(A²-Y⁴)ₒ-Z
sind, in der die Variablen die oben genannten Bedeutungen haben.

5. Verwendung von chiralen Verbindungen der allgemeinen Formel Ia 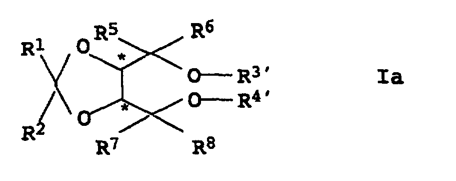 wobei die Variablen die folgende Bedeutung haben:
R¹,R²,R^{3'},R^{4'} Wasserstoff oder aromatische, aliphatische oder araliphatische Reste mit 1 bis 40 C-Atomen,
R⁵,R⁶,R⁷,R⁸ aromatische oder aliphatische Reste mit 1 bis 20 C-Atomen,
als Dotierstoffe in Flüssigkristallen.

6. Flüssigkristalline Zusammensetzungen, enthaltend als Dotierstoff eine oder mehrere chirale Verbindungen der Formel I oder Ia gemäß den Ansprüchen 1 bis 5.

7. Flüssigkristalline Zusammensetzungen nach Anspruch 6, enthaltend zusätzlich zu den Verbindungen I oder Ia polymerisierbare achirale flüssigkristalline Verbindungen.

8. Verwendung der flüssigkristallinen Zusammensetzungen gemäß den Ansprüchen 6 oder 7 zur Beschichtung von Substraten.

9. Verwendung der flüssigkristalline Zusammensetzungen gemäß den Ansprüchen 6 oder 7 zur Herstellung von Interferenzpigmenten.

10. Verwendung der Pigmente nach Anspruch 9 in Druckfarben und Lacken.

## Claims

1. A chiral compound of the formula I 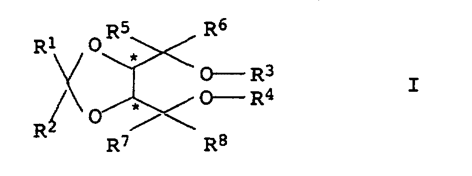 where
R¹ and R² are hydrogen or aromatic, aliphatic or araliphatic radicals having 1 to 40 carbon atoms,
R³ and R⁴ are aromatic, aliphatic or araliphatic radicals having 1 to 40 carbon atoms,
R⁵,R⁶,R⁷ and R⁸ are aromatic or aliphatic radicals having 1 to 20 carbon atoms,
with the proviso that at least one of the radicals R¹ to R⁸ contains a polymerizable group.

2. A chiral compound of the formula I as claimed in claim 1, where R⁵, R⁶, R⁷ and R⁸ are unsubstituted or vinyl- or vinyloxy-substituted phenyl or naphthyl radicals.

3. A chiral compound of the formula I as claimed in claim 1 or 2, where R³ and R⁴ are,
Y¹-(A¹-Y²)ₘ-(T-Y³)ₙ-(A²-Y⁴)ₒ-Z
where
Y¹ is a single covalent bond, -CO- or -CO-O-,
Y², Y³ and Y⁴ are a single covalent bond, oxygen, sulfur, -CO-O-, -O-CO-, -O-CO-O-, -CO-NR⁹- or -NR⁹-CO-, where these bridges may be identical or different,
A¹ and A² are identical or different alkylene radicals having 2 to 12 carbon atoms, in which non-terminal, non-adjacent CH₂ groups may be replaced by oxygen,
T are identical or different aliphatic or aromatic rings, which may contain up to 3 hetero atoms,
R⁹ is hydrogen or C₁-C₄-alkyl,
m and o are 0 or 1,
n is 0, 1, 2, 3 or 4, and
Z is hydrogen or a polymerizable radical.

4. A chiral compound of the formula I as claimed in claims 1 to 3, where R¹ and R², independently of one another are hydrogen or radicals of the formula
-(A¹-Y²)ₘ-(T-Y³)ₙ-(A²-Y⁴)ₒ-Z
where the variables are as defined above.

5. The use of a chiral compound of the formula Ia 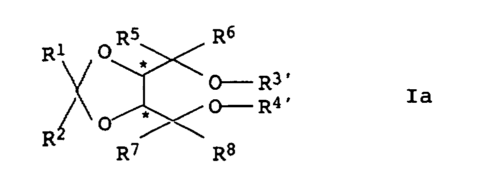 where:
R¹,R²,R^{3'} and R^{4'} are hydrogen or aromatic, aliphatic or araliphatic radicals having 1 to 40 carbon atoms,
R⁵,R⁶,R⁷ and R⁸ are aromatic or aliphatic radicals having 1 to 20 carbon atoms,
as dopes in liquid crystals.

6. A liquid-crystalline composition containing, as dope, one or more chiral compounds of the formula I or Ia as claimed in claims 1 to 5.

7. A liquid-crystalline composition as claimed in claim 6, containing polymerizable, achiral liquid-crystalline compounds in addition to the compounds of the formula I or Ia.

8. The use of a liquid-crystalline composition as claimed in claim 6 or 7 for coating substrates.

9. The use of a liquid-crystalline composition as claimed in claim 6 or 7 for the preparation of interference pigments.

10. The use of a pigment as claimed in claim 9 in printing inks and surface coatings.

## Revendications

1. Composés chiraux de formule générale I 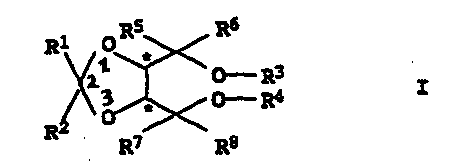 dans laquelle les variables prennent la signification suivante :
R¹,R² représentent un atome d'hydrogène, des résidus aromatiques, aliphatiques ou araliphatiques ayant 1 à 40 atomes de C,
R³,R⁴ représentent des résidus aromatiques, aliphatiques ou araliphatiques ayant 1 à 40 atomes de C.
R⁵,R⁶,R⁷,R⁸ représentent des résidus aromatiques, ou aliphatiques ayant 1 à 20 atomes de C,
avec la condition qu'au moins un des résidus R¹ à R⁸ contienne un groupe polymérisable.

2. Composés chiraux I selon la revendication 1, dans lesquels R⁵, R⁶, R⁷ et R⁸ sont des résidus phényle ou naphtyle non substitués ou substitués par des groupes vinyle ou vinyloxy.

3. Composés chiraux I selon la revendication 1 ou 2, dans lesquels R³ et R⁴ sont des résidus de formule générale,
-Y¹-(A¹-Y²)ₘ-(T-Y³)ₙ-(A²-Y⁴)ₒ-Z
dans laquelle les variables prennent la signification suivante :
Y¹ représente une simple liaison covalente, -CO- ou-CO-O-,
Y²,Y³,Y⁴ représentent une simple liaison covalente, un atome d'oxygène, un atome de soufre, -CO-O-, -O-CO-, -O-CO-O-, -CO-NR⁹- ou NR⁹-CO-, ces éléments de pont pouvant être identiques ou différents,
A¹,A² représentent des résidus alkylène identiques ou différents ayant 2 à 12 atomes de C, dans lesquels les groupes CH₂ non terminaux, non vicinaux peuvent être remplacés par un atome d'oxyqène,
T représente des cycles aromatiques ou aliphatiques, identiques ou différents, qui peuvent contenir jusqu'à trois hétéroatomes,
R⁹ représente un atome d'hydrogène ou un groupe alkyle en C₁ à C₄,
M, o 0 ou 1,
n vaut 0, 1, 2, 3 ou 4 et
Z un atome d'hydrogène ou un résidu polymérisable.

4. Composés chiraux I selon les revendications 1 à 3, dans lesquels R¹ et R² représentent indépendamment l'un de l'autre un atome d'hydrogène ou des résidus de formule générale
- (A¹-Y²)ₘ- (T-Y³)ₙ-(A²-Y⁴)ₒ-Z
dans laquelle les variables prennent les significations précitées.

5. Utilisation des composés chiraux de formule générale Ia 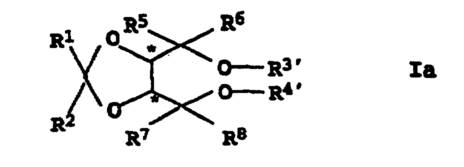 dans laquelle les variables prennent la signification suivante :
R¹,R²,R³,R⁴ représentent un atome d'hydrogène, des résidus aromatiques, aliphatiques ou araliphatiques ayant 1 à 40 atomes de C,
R⁵,R⁶,R⁷,R⁸ représentent des résidus aromatiques ou aliphatiques ayant 1 à 20 atomes de C,
en tant que substances dopantes dans les cristaux liquides.

6. Compositions à cristaux liquides, contenant comme substance dopante un ou plusieurs composés chiraux de formule générale I ou Ia selon les revendications 1 à 5.

7. Compositions à cristaux liquides, selon la revendication 6 contenant en plus des composés I ou Ia des composés à cristaux liquides achiraux polymérisables.

8. Utilisation des compositions à cristaux liquides selon les revendications 6 ou 7, pour revêtir des substrats.

9. Utilisation des compositions à cristaux liquides selon les revendications 6 ou 7, pour préparer des pigments interférentiels.

10. Utilisation des pigments selon la revendication 9, dans les encres d'impression et les laques.
